(19) Europäisches Patentamt
European Patent Office
Office européen des brevets

(11) **EP 3 226 949 B1**

(12) **EUROPÄISCHE PATENTSCHRIFT**

(45) Veröffentlichungstag und Bekanntmachung des Hinweises auf die Patenterteilung:
**21.08.2019 Patentblatt 2019/34**

(21) Anmeldenummer: **15834755.9**

(22) Anmeldetag: **04.12.2015**

(51) Int Cl.:
*A61M 16/04* (2006.01)    *A61M 25/10* (2013.01)

(86) Internationale Anmeldenummer:
**PCT/IB2015/002309**

(87) Internationale Veröffentlichungsnummer:
**WO 2016/087930 (09.06.2016 Gazette 2016/23)**

(54) **VORRICHTUNG ZUR DYNAMISCH DICHTENDEN OKKLUSION ODER RAUMFULLENDEN TAMPONADE EINES HOHLORGANS**

DEVICE FOR THE DYNAMICALLY SEALING OCCLUSION OR SPACE-FILLING TAMPONADE OF A HOLLOW ORGAN

DISPOSITIF PERMETTANT DE RÉALISER L'OCCLUSION D'UN ORGANE CREUX EN ASSURANT UNE ÉTANCHÉITÉ DYNAMIQUE OU LE TAMPONNEMENT D'UN TEL ORGANE PAR REMPLISSAGE D'ESPACE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: 04.12.2014 DE 102014017872
22.01.2015 DE 102015000621
29.01.2015 DE 102015001030
04.03.2015 DE 102015002995
18.11.2015 DE 102015014824

(43) Veröffentlichungstag der Anmeldung:
**11.10.2017 Patentblatt 2017/41**

(73) Patentinhaber: **Creative Balloons GmbH**
**68753 Waghäusel (DE)**

(72) Erfinder: **GÖBEL, Fred**
**69259 Wilhelmsfeld (DE)**

(74) Vertreter: **Küchler, Stefan**
**Patentanwalt**
**Färberstrasse 20**
**90402 Nürnberg (DE)**

(56) Entgegenhaltungen:
US-A- 4 762 129      US-A- 5 029 591
US-A- 5 947 927      US-A1- 2007 277 830
US-A1- 2013 146 062

**Beschreibung**

Die Erfindung richtet sich auf eine Vorrichtung zur dynamischen Okklusion bzw. Tamponade eines Hohlorgans mittels eines ballonartigen Elementes, insbesondere zur dynamischen, aspirationspräventiven Dichtung der intubierten Trachea bei eigenständig atmenden Patienten sowie bei Patienten in einem maschinell unterstützten Spontanatmungsmodus.

Ein grundsätzliches Problem beim organverträglich und effizient dichtenden Verschluss bzw. bei der raumfüllenden Tamponade von Organen oder Kavitäten mit einem befüllbaren, ballonartigen Element, ist die in vielen Fällen fortwährend wirkende Motilität des Organs selbst. Muskulös-bindegewebig begrenzte Organe oder Körperhöhlen weisen häufig eine eigenständige Motilität auf oder sind der Dynamik angrenzender Organe oder Strukturen ausgesetzt. Derartig eigen- oder korrespondierend-motile Organe erfordern für einen kontinuierlich wirkenden Verschluss des Organlumens einen besonderen, rasch auf Schwankungen des Organdurchmessers bzw. Änderungen des Tonus der Organwandung reagierenden Regelmechanismus. Der Mechanismus muss möglichst zeitsynchron zur eintretenden Durchmesser- oder Tonusveränderung im Organ wirken.

Das Problem der dynamischen, zeitsynchronen Anpassung einer Ballonokklusion eines Hohlorgans kann am Beispiel der menschlichen Luftröhre verbildlicht werden. Die Luftröhre (Trachea) ist eine aus knorpeligen, bindegewebigen und muskulösen Anteilen aufgebaute rohrartige Struktur. Sie reicht vom unteren Abschnitt des Kehlkopfes bis zur Verzweigung in die Hauptbronchien. Die vordere und die seitlichen Portionen der Luftröhre sind dabei durch spangenartige, in etwa hufeisenförmige Strukturen stabilisiert, die wiederum durch bindegewebeartige Gewebelagen in Längsrichtung miteinander verbunden sind. Auf der rückwandigen Seite wird das Luftröhrenlumen durch die sogenannte Pars membranacea abgeschlossen, die aus durchgängig muskulös-bindegewebigem Material, ohne versteifende knorpelige Elemente besteht. Ihr liegt dorsal wiederum die muskulösbindegewebige Speiseröhre an.

Das obere Drittel der Trachea befindet sich in der Regel außerhalb des Brustkorbes (Thorax), während die beiden unteren Drittel innerhalb der von Brustkorb und Zwerchfell abgegrenzten Brusthöhle liegen. Der untere, thorakale Anteil der Trachea ist so in besonderer Weise den Druckschwankungen in der Brusthöhle ausgesetzt, die sich im Rahmen der thorakalen Atemmechanik eines spontan atmenden oder auch assistiert spontan atmenden Patienten einstellen.

Bei der Einatmung des Patienten wird das thorakale Volumen durch die Hebung der Rippen und simultane Senkung des Zwerchfells vergrößert, wodurch der intrathorakale Druck abfällt. Dieser Druckabfall führt atemmechanisch zum Einströmen von Atemluft, in die sich durch die thorakale Volumenvergrößerung erweiternde Lunge.

Der mit der thorakalen Volumenvergrößerung einhergehende Druckabfall im Thorax führt allerdings ebenfalls zu korrespondierenden Druckabfällen innerhalb von befüllten Ballonelementen die im Thorax des Patienten positioniert sind. Solche Ballonelemente werden zum Beispiel bei Trachealtuben und Tracheostomiekanülen verwendet, um die tiefen Luftwege gegen einströmende Sekrete des Rachens zu dichten sowie um eine positive Druckbeatmung der Patientenlunge zu ermöglichen. Die zyklischen, durch die Patienteneigenatmung erzeugten thorakalen Druckabfälle können den dichtungswirksamen Druck in den Ballons von Beatmungskathetern in niedrige Bereiche bewegen, bei denen eine suffiziente Dichtung gegen Sekrete, die sich oberhalb des dichtenden Ballons (Cuff) in der Luftröhre sammeln, nicht mehr gewährleistet ist. Siehe hierzu, Badenhorst CH, Changes in tracheal cuff pressure in respiratory support, CritCareMed, 1987; 15/4: 300-302.

Während die Dichtungsleistung herkömmlicher Trachealtuben-Cuffs aus PVC sehr eng mit dem jeweils im Cuff wirkenden Fülldruck korreliert, zeigen besonders dünnwandige Trachealtuben-Cuffs aus Polyurethan eine deutlich stabilere Dichtungseffizienz, wenn die tracheal wirkenden Dichtungsdrucke in Bereiche um 15 mbar abfallen. Siehe Bassi GL, CritCareMed, 2013; 41: 518-526.

Von besonderer Bedeutung für die Sekretdichtung trachealer Dichtungsballons ist jedoch insbesondere der Druckbereich von 5 bis 15 mbar, der bei der Eigenatmung des Patienten, quasi von Atemhub zu Atemhub, in vielen Fällen problemlos erreicht wird. Dichtungsoptimierte Beatmungskatheter mit mikrodünnwandigen PUR-Cuffs vermögen zwar auch hier noch bei ca. 10 mbar Fülldruck gute Dichtungsleistungen zu gewährleisten, können bei zu Druckabfällen unter 10 mbar, bis auf subatmosphärische, thorakale Druckwerte jedoch nicht gegen einströmendes, infektiöses Sekret schützen.

Eine der der aktiven Atemarbeit des Thorax zeitsynchron folgende, atraumatische, über ausreichend weite Fülldruckbereiche dichtungseffizient wirkende und kostengünstig herstellbare Verschlusstechnik der Luftröhre mittels eines cuff-artigen Dichtungsballons ist bislang nicht verfügbar. Zwar sind im Stand der Technik verschiedenste technische Ausführungen fülldruckregulierender Vorrichtungen für tracheale Beatmungskatheter beschrieben, eine ausreichend zeitsynchrone Anpassung des Dichtungsdrucks an alternierende thorakale Drucke, wie sie bei der Eigenatmung des Patienten beobachtet werden, ist bislang jedoch nicht erreicht.

Bei den bekannten Beatmungskathetern erfolgt die Befüllung des tracheal dichtenden Ballonelementes in der Regel durch kleinlumige, in den Schaft des Katheters hinein extrudierte Befüllzuleitungen. Die niedrigen Querschnitte der Befüll-Leitungen gewährleisten in der Regel keinen ausreichend großen Volumenstrom des den Ballon dichtenden Füll-Mediums, um eine die tracheale Dichtung während eines thorakalen Druckabfalls aufrechtzuerhalten. Selbst technisch aufwendige extrakorporale Regelmechanismen, wie beispielsweise das Gerät CDR 2000 der Firma Logomed GmbH (nicht mehr im Handel), sind durch die Kleinlumigkeit der Zuleitung zwischen dichtendem Ballonelement und Regler in ihrer Funktion nur unzureichend.

Zu den relevanten Stand der Technik gehören: US4762129 dass offenbart einen Dehnungskatheter mit einer aufblasbaren Manschette. US 213/146062 A1 offenbart eine Manschette mit unterschiedlichen Außendurchmessern. US 5029591 A offenbart einen Endotrachealtubus mit einem Sekundärlumen und auch ein Rückschlagventil in dem Sekundärlumen. US 2007/277830 A1 offenbart ein Intubationsschlauch mit einer Manschette zum Abdichten der Atemwege eines Patienten.

Die vorliegende Erfindung beschreibt eine neuartige Kathetertechnik, die durch eine besonders fluss-effizient gestaltete Zuleitung des Füllmediums einen raschen Volumenstrom zwischen einem körper-externen Regelmechanismus und einem in der Trachea platzierten, ballonartigen Dichtungselement ermöglicht. Der Umfang der Erfindung richtet sich nach den zugehörigen Ansprüchen. Es wird so, auf technologisch einfache und kostengünstige Weise, eine für die dynamische Dichtung der Trachea eins spontanatmenden Patienten ausreichend zeitsynchrone, die Dichtung aufrechterhaltende Kompensation des Cuffvolumens erreicht.

Die Erfindung beschreibt ferner einen dünnwandigen, einlumig ausgeführten Schaft eines Beatmungskatheters, dessen Schaftwandung bevorzugt durch eine wellschlauch-artige Korrugation stabilisiert ist. Die Korrugation des Schaftes ermöglicht so besonders niedrige Wandstärken, was optimal große Innenlumina für die besonders widerstandsarme Atmung des Patienten ermöglicht. Die Korrugation verleiht dem Schaft zudem eine besondere, spannungslose bzw. nicht elastisch wirkende, axiale Verbiegbarkeit und damit Anpassungsfähigkeit in der Trachea eines sich spontan bewegenden Patienten. Ein derart flexibler Schaft kann zudem aus besonders harten PUR-Typen hergestellt werden, die wiederum besonders niedrige Schaftwandstärken ermöglichen.

Eine mögliche Ausführungsform der Erfindung beruht auf ein Ballonelement, welches am distalen Ende des Beatmungskatheters fest und dicht schließend auf dem tragenden Schaftkörper aufgebracht ist und dessen proximales Ende sich annähernd auf das Außenmaß des Katheterschaftes verschlankt, jedoch einen koaxialen Spaltraum zum Schaft bestehen lässt. Diese proximale Verlängerung des Ballons kann in den thorakalen Abschnitt der Luftröhre reichen, aber auch in den extra-thorakalen Abschnitte der Trachea, bis in den Bereich der Stimmlippen oder auch den Bereich des unteren Rachens geführt sein, wobei sich wiederum zwischen der Hülle des so verschlankten proximalen Ballonsegmentes und dem umschlossenen Schaft ein bestimmter freier Spaltraum einstellt.

Der so geschaffene Spaltraum gestattet einen besonders großlumigen, effizient wirkenden Volumenfluss zwischen einem außerhalb des Körpers angeordneten Volumenreservoir und dem tracheal dichtenden Ballonelement. Der Spalt verhindert ferner den potentiell schädigenden, direkten Kontakt des korrugierten Katheterschaftes mit dem Epithel der Luftröhre.

Die konzentrische Anordnung des Katheterschaftes in einem verjüngten schlauchartigen proximalen Ballonsegment ist insbesondere im Bereich der Stimmlippen von Vorteil. Die schaftumschließende Ballonhülle liegt hier den Stimmlippen schützend an und vermeidet abrasive Wirkungen des Schaftes bei Relativbewegungen des Schaftes zum Atemtrakt.

Im proximalen Abschnitt des trachealen Beatmungskatheters wird das Ende des schlauchartig verjüngten Ballonsegmentes bevorzugt durch ein mehrlumiges Schaftelement aufgenommen. Dieses Schaftelement weist bevorzugt einen Zuleitungsquerschnitt auf, dessen flusswirksamer Querschnitt vorzugsweise dem fluss-wirksamen Querschnitt des Spaltraumes zwischen Schaft und proximalem Ballonsegment entspricht. An das in den proximalen Katheter intergrierte Zuleitungslumen schließt sich dann wiederum ein Befüllschlauch an, der einen Durchmesser mit entsprechenden Fluss-Charakteristika aufweist, wie sie innerhalb des Katheters gewährleistet sind.

Die Erfindung zeigt beispielhaft einen Berechnungsweg für die Dimensionierung des erfindungsgemäß geschaffenen Spaltraumes zwischen dem Katheterschaft und der proximalen Verlängerung des Ballonendes bzw. Ballonkorpus, der in guter Näherung aufzeigt, wie groß der radiale Spalt sein muss um in weniger als 10 Millisekunden nach dem Beginn einer thorakalen Drucksenkung einen ausreichend kompensierenden Volumenfluss zwischen Regler und Ballon zu erreichen.

Sowohl das den Schaft umschließende proximale Ballonsegment, als auch das distale, tracheal dichtende Ballonsegment, werden bevorzugt aus einem möglichst dünnwandigen, nur wenig volumendehnbaren Material hergestellt. Hierdurch wird die erforderliche Formstabilität des Ballonkörpers bei Belastung mit Fülldruck von Innen oder bei mechanischer Belastung von außen gewährleistet.

Die Erfindung beschreibt ferner verschiedene Verfahren zur rasch volumenkompensierenden Dichtung der Trachea, wobei ein erfindungsgemäßer, mit einem okkludierenden oder tamponierenden Ballonelement versehener Katheter an eine extrakorporale Reglervorrichtung angeschlossen ist. Durch die Kopplung des Katheters mit dem Regler entsteht ein mit isobarem Fülldruck beaufschlagter Binnenraum.

Die durchgängig fluss-effizient gestaltete, großlumige Verbindung zwischen dem dichtenden Ballonelement und einer druckkonstant gehaltenen Volumenquelle, stellt die erforderliche Synchronizität zwischen der thorakalen Atemtätigkeit des Patienten und dem zur Erhalt der trachealen Dichtung erforderlichen Volumenfluss her.

Rasche Volumenverschiebungen werden im erfindungsgemäßen Verbund von Katheter und Regler beispielsweise durch schwerkraft- oder federkraftangetriebene Regler ermöglicht, die im Bereich niedriger, physiologisch unbedenklicher Drucke, also einem Druckbereich von ca. 25 bis 35 mbar arbeiten und zur Gewährleistung ausreichender Volumenflüsse über die verbindende Zuleitung zwischen Cuff und Regler keine den Volumenfuß forcierende, unphysiologisch hohe Druckgradienten benötigen.

Der Regler kann beispielsweise in der in PCT/EP/2013/056169 beschriebenen Bauart ausgeführt werden. Die vorliegende Erfindung ist bevorzugt für diese einfache Form der Regelung durch ein kommunizierendes System von Binnenräumen unter isobarem Druck im physiologischen Druckbereich von 20 bis 50, bevorzugt 20 bis 35 mbar, ausgelegt.

Der erfindungsgemäß optimierte Volumenfluss zwischen tracheal dichtendem Ballonkörper und extrakorporalem Regler kann auch bei Trachealtuben und Tracheostomiekanülen konventioneller Bauart erreicht werden, sofern der zuleitende Füllkanal eine flusswirksame Quersschnittfläche aufweist, die der einer kreisrunden Querschnittsfläche bei einem Kreisdurchmesser von mindestens 2 mm, bzw. bevorzugt einem Kreisdurchmesser von 4 bis 6 mm entspricht. Bei Produkten üblicher Bauart ist der volumenzuführende Kanal in der Regel kreisrund und weist einen Innen-Durchmesser von ca. 0,5 bis 0,7 mm auf. Der Kanal ist in die Schaftwandung des Tubenschaftes extrudiert. Er geht außerhalb des Schaftes in eine Schlauchleitung über, die in der Regel mit einem Einwegeventil abgeschlossen ist. Im Sinne möglichst dünnwandiger Schäfte bzw. um möglichst große Innendurchmesser des Beatmungslumens zu ermöglichen, werden die Zuleitungen in der Schaftwandung entsprechend niedrig-kalibrig gehalten.

Das beschriebene Prinzip der fluss-optimierten Volumenkompensation kann in analoger Weise bei der dynamischen Tamponade, der Speiseröhre eines spontan atmenden Patienten zur Anwendung kommen. Die zur Eigenatmung korrespondierenden Druckschwankungen in einem ösophageal platzierten Tamponadeballon sind in der Regel ausgeprägter als bei einem tracheal platzierten Ballon. Sie entsprechen in der Regel dem jeweils herrschenden, absoluten intra-thorakalen Druck. Zur Herstellung einer möglichst effizient dichtenden Ballontamponade in der Speiseröhre wird eine Segmentierung des Ballons vorgeschlagen, die der in Trachea entspricht. Während das eigentlich ösophageal dichtende, distale Segment des Ballons sich über die Ausdehnung der Speiseröhre zwischen deren oberen und unteren Sphinkter erstreckt, schließt sich nach proximal ein verjüngtes Ballonsegment an, welches optional bis zum proximalen Ende des den Ballon tragenden Katheter reicht. Die im Folgenden am Beatmungskatheter dargestellten, den resultierenden Spaltraum zwischen Schaft und proximalem Ballonsegment definierenden Durchmesserverhältnisse sind auch im Falle von Magensonden für die Ernährung und/oder Dekompression anwendbar. Auch hier kann der Schaft der Magensonde aus einem einlumigen, dünnwandigen Schlauch bestehen, der zur Verbesserung der Biegemechanik ganz oder in Anteilen korrugiert ist. Die Anbindung an ein isobar wirkendes Volumenreservoir kann in erfindungsgemäßer Weise analog zum Trachealtubus erfolgen.

Weitere Merkmale, Einzelheiten, Vorteile und Wirkungen auf der Basis der Erfindung ergeben sich aus der folgenden Beschreibung bevorzugter Ausführungsbeispiele der Erfindung sowie anhand der zugehörigen Zeichnungen.

Fig. 1    zeigt einen erfindungsgemäßen Trachealtubus im Verbund mit einer externen, volumenregulierenden Vorrichtung.

Fig. 2    zeigt den volumenverschiebenden Spalt S in Bezug zum Gesamtdurchmesser G im Bereich der proximalen Ballonverlängerung.

Fig. 3    zeigt einen Trachealtubus mit mehreren, im oder am Katheterschaft angeordneten volumenverschiebenden Zuleitungen.

Fig. 3a    zeigt die zuleitenden, schaftintegrierten Lumina des in Fig. 3 beschriebenen Tubus im Querschnitt.

Fig. 4    zeigt eine besondere Ausführung des in Fig.1 gezeigten Tubus, dessen tracheal dichtender Ballon über die Glottisebene hinausreicht.

Fig. 5    zeigt eine beispielhafte Ausführungsform einer erfindungsgemäßen Tracheostomiekanüle.

Fig. 6    zeigt einen erfindungsgemäßen Trachealtubus mit einem Sensorelement im Bereich des tracheal dichtenden Ballonsegmentes und einer mit dem Sensor in einem Regelkreis angeordneten Reglereinheit.

Fig. 7    zeigt einen kombinierten Ventil-/Drosselmeachanismus, der die rasche dichtungskritische Entleerungen des Ballons zum Regler/Reservoir hin vermeidet.

Fig. 8    zeigt eine Magensonde mit nach proximal verlängertem Ballonsegment, für die dynamisch dichtende Tamponade der Speiseröhre im Verbund mit einem extrakorporalen, isobaren Volumenreservoir.

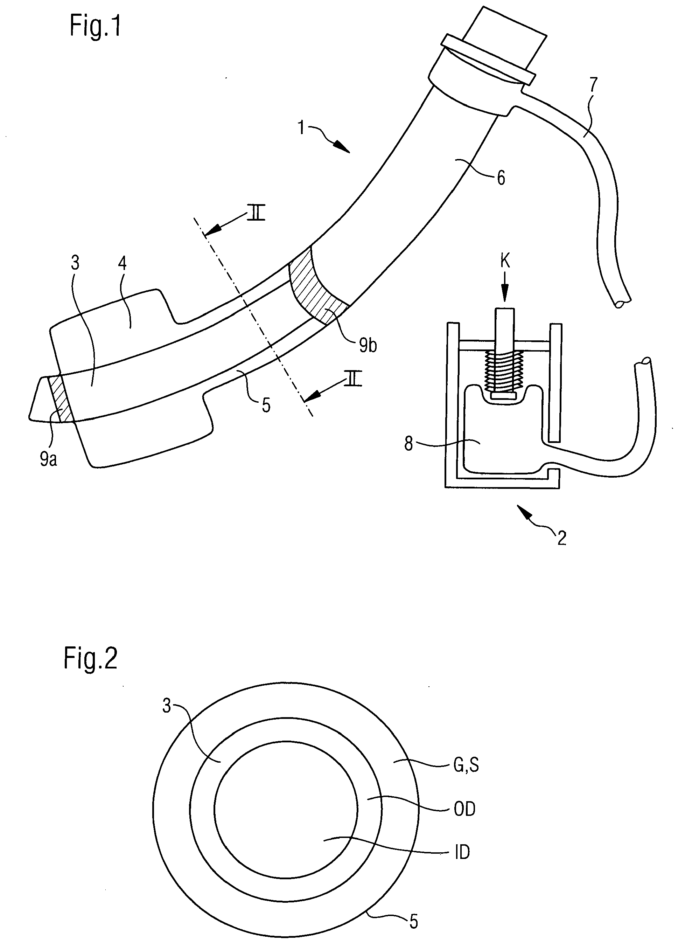

Fig. 1 beschreibt in einer beispielhaften Gesamtübersicht die kommunizierende Verbindung/Kopplung einer erfindungsgemäßen Vorrichtung 1 in Form eines Trachealtubus mit einem vorzugsweise schwerkraft- oder federkraft-angetriebenen, isobar wirkenden Volumenreservoir 2.

Der durch die frei kommunizierende Kopplung von Trachealtubus und Volumenreservoir 2 entstehende Zuleitungsraum besteht aus dem tracheal dichtenden Ballonsegment 4, dem sich nach proximal anschließenden, schlauchartig verjüngten Ballonende 5, dem Zuleitungslumen bzw. den Zuleitungslumina im proximalen Schaftelement 6, der sich an den Schaft anschließenden Schlauch-Zuleitung 7 zum Reservoir sowie dem Reservoir-Volumen 8 des Reglers.

Der distale Schaftanteil des Tubus 3 trägt an seinem distalen Ende einen tracheal dichtenden Ballon 4, welches mit seinem distalen Ende 9a mit der Oberfläche des Schaftes dicht schließend verbunden ist. Nach proximal schließt sich an das Ballonsegment 4 ein relativ zum Durchmesser des dichtenden Ballonsegmentes 4 verjüngtes, schlauchartiges proximales Ballonsegment 5 an. Sein proximales Ende schließt dicht mit der Oberfläche des distalen Endes 9b des proximalen Schaftelementes 6 ab.

Kommt es im Verlauf der Einatmung (Inspiration) zu einer Absenkung des intra-thorakalen Druckes, und somit zu einer korrespondierenden, passageren Weitung des trachealen Querschnittes, welcher wiederum einen korrespondierenden Druckabfall innerhalb des tracheal platzierten Ballonkorpus verursacht, strömt Volumen, vom Reservoir 2 zum dichtenden Ballonsegment 4, wobei das Reservoir das Volumen fortwährend mit einem definierten Druck beaufschlagt hält. Hierdurch kann der tracheale Dichtungsdruck auch im Falle einer tiefen Inspiration des Patienten, mit einem eventuellen Druckabfall im Thorax bzw. im tracheal dichtenden Ballon auf subatmosphärische Werte, ohne relevante Einbuße des Dichtungsvermögens aufrechterhalten werden.

In bevorzugter Ausführung besteht das Reservoir 2 aus einem Reservoirkörper 8, welcher beispielsweise als Ballon oder Balg ausgebildet sein kann und durch eine auf das Reservoir wirkende Kraft K einen fortwährend wirkenden isobaren Druck im Zuleitungsraum einstellt.

Entscheidend für die kleinstmögliche zeitliche Latenz zwischen einer beginnenden Weitung des trachealen Querschnittes bzw. einer beginnenden Reduktion der transmural auf den tracheal platzierten Dichtungsballon wirkenden thorakalen Kräfte und einer einsetzenden, dichtungswirksamen Verschiebung von Füllmedium zum tracheal dichtenden Ballonsegment, ist vor allem die fluss-wirksame Querschnittfläche des zwischen dem distalen Schaftanteil 9 und der proximalen Verlängerung 5 des Ballons verfügbaren Spaltraums S.

Die Erfindung schlägt in der folgenden Figur besonders vorteilhaft dimensionierte Verhältnisse der Querschnittfläche S zur Querschnittfläche des Beatmungslumens ID sowie zur Gesamtquerschnittsfläche OD des Katheters zwischen dem proximalen Schaft 6 und dem zum tracheal dichtenden Ballonsegmente 4 vor.

Fig. 2 zeigt einen Querschnitt durch das sich nach proximal an den tracheal dichtenden Ballon anschließende, volumenzuleitende Segment 5 des Ballons, des in Fig. 1 dargestellten Trachealtubus.

S bezeichnet die für die Zuleitung von Füllmedium zum Ballon bevorzugte Spaltfläche. Sie definiert sich als Differenz zwischen der durch die Hüllenwandung des zuführenden Ballonendes 5 begrenzten Querschnittfläche G und der durch die Schaftaußenfläche begrenzten Querschnittfläche OD des Schaftkörpers. Die Querschnittfläche S soll dabei 1/10 bis 5/10 der Querschnittsfläche G, besonders bevorzugt 2/10 bis 3/10 der Querschnittsfläche G betragen.

Bezogen auf die Querschnittsfläche ID des Innenlumens des Schaftkörpers soll die Querschnittfläche S 2/10 bis 6/10 der Querschnittsfläche ID, besonders bevorzugt 3/10 bis 4/10 der Querschnittsfläche ID betragen.

Neben Luft als bevorzugtem Medium können auch flüssige Medien zur Befüllung des tracheal dichtenden Systems verwendet werden.

Für die quantitative Berechnung der Strömungsverhältnisse in volumenleitenden Ballonbinnenraum, insbesondere auf die Druckverhältnisse im tracheal dichtenden Ballonsegment 4, sollen folgende Platzhaltergrößen verwendet werden:

$V_1$ Volumen des distalen Ballonsegmentes 4
$p_1$ Druck im distalen Ballonsegment 4
$\rho_1$ Fülldichte im distalen Ballonsegment 4
$M_1$ Luftmasse im distalen Ballonsegment 4
$V_2$ Volumen des extrakorporalen Reservoirs 8
$P_2$ Druck im extrakorporalen Reservoir 8
$\rho_2$ Fülldichte im extrakorporalen Reservoir 8
$m_2$ Luftmasse im extrakorporalen Reservoir 8

Für die Luftmassen $m_1$, $m_2$ gilt:

$$m_1(t) = m_{1,0} + \int_{\tau=0}^{\tau=t} S_{m,1}(\tau)\, d\tau \qquad (1)$$

$$m_2(t) = m_{2,0} - \int_{\tau=0}^{\tau=t} S_{m,2}(\tau)\, d\tau \qquad (2)$$

Dabei meint $S_{m,v}$ die Luftströmung zu dem betreffenden Ballon 4, 8 als Luftmassenströmung.

Gemäß dem Gesetz von Hagen-Poiseuille gilt für die massenbezogene Fluidströmung durch eine Leitung mit kreisrundem Querschnitt und dem Innenradius R und einer Länge l:

$$S_{m,1} = \frac{\rho_1 \cdot \pi\,(p_2 - p_1) \cdot R^4}{8\,\eta \cdot l} \qquad (3a)$$

$$S_{m,2} = \frac{\rho_2 \cdot \pi\,(p_1 - p_2) \cdot R^4}{8\,\eta \cdot l} \qquad (3b)$$

Wenn jedoch - wie hier - das sekundäre Lumen eine ringförmige Struktur um ein primäres Lumen herum darstellt, so trifft die Formel von Hagen-Poiseuille nicht exakt zu.

Man muss stattdessen einen Raum mit einem streifenförmigen Querschnitt betrachten, den man sich idealerweise begradigt vorstellen kann, also von ebener Struktur bzw. mit einem rechteckigen Querschnitt der Länge L und der Dicke D, also mit einer Querschnittsfläche $Q = L \cdot D$.

Zwischen zwei Platten mit einem Abstand D gilt für die Verteilung der Strömungsgeschwindigkeit v(x) entlang einer zu den Platten lotrechten Richtung x:

$$v(x) = \frac{(p_1 - p_2) \cdot (D^2/4 - x^2)}{2\,\eta \cdot l} \qquad (4)$$

Dies ist eine parabelförmige Kurve. Durch Integration über die Querschnittsfläche Q lässt sich die durch die Querschnittsfläche Q in der Zeit t strömende Masse ermitteln zu:

$$S_{m,1} = \frac{\rho_1 \cdot (p_2 - p_1) \cdot L \cdot D^3}{12\,\eta \cdot l} \qquad (5a)$$

$$S_{m,2} = \frac{\rho_2 \cdot (p_1 - p_2) \cdot L \cdot D^3}{12\,\eta \cdot l} \qquad (5b)$$

Diese Formeln ersetzen jedenfalls für den ringförmigen Ballonabschnitt 5 die obigen Formeln (3a) und (3b) von Hagen-Poiseuille

Dabei ist $\eta$ die dynamische Viskosität des strömenden Gases. Für Luft gilt:

$\eta$ beträgt bei 273 K 17,1 $\mu$Pa $\cdot$ s

Ferner gilt aufgrund der thermischen Zustandsgleichung idealer Gase im Ballon 4:

$$\eta_1 = \rho_1 \cdot R_S \cdot T_1 \qquad\qquad (6a)$$

und im Ballon 8:

$$\eta_2 = \rho_2 \cdot R_S \cdot T_2 \qquad\qquad (6b)$$

Dabei ist $R_S$ die individuelle oder spezifische Gaskonstante, welche für Luft den Wert 287,058 J/(kg$\cdot$K) hat.

$T_v$ ist die Temperatur in den Ballonabschnitten 4 bzw. 5 bzw. in dem Ballon 8.

Für eine Temperatur von 23 °C bzw. 296 K beträgt der Faktor

$$k = R_{S,Luft} \cdot T_{23^oC} = 85 \cdot 10^3 \text{ J/(kg}\cdot\text{K)} \qquad\qquad (7)$$

Im Folgenden soll angenommen werden, dass die Temperatur sowohl in dem Ballon 4 als auch im Ballon 8 konstant 23° C beträgt:

$$T_1 = T_2 = 296 \text{ K.}$$

Dann gilt:

$$p_1 = \rho_1 \cdot k \qquad\qquad (8)$$

$$p_2 = \rho_2 \cdot k \qquad\qquad (9)$$

Damit ergibt sich durch Einsetzen von Gleichung (5a) in Gleichung (1):

$$m_1(t) = m_{1,0} + \int_{\tau=0}^{\tau=t} \frac{\rho_1 \cdot L \cdot D^3}{12\,\eta \cdot l}\,(p_2 - p_1)\,d\tau \qquad\qquad (10)$$

Mit der Gleichung (8) folgt daraus:

$$m_1(t) = m_{1,0} + \int_{\tau=0}^{\tau=t} \frac{\rho_1 \cdot L \cdot D^3}{12\,\eta \cdot l \cdot k}\,p_1\,(p_2 - p_1)\,d\tau \qquad\qquad (11)$$

Ferner gilt im Ballon 4 :

$$\frac{m_1}{V_1} = \rho_1 = \frac{p_1}{k} \qquad (12)$$

Daher lässt sich in Gleichung (11) für die Masse $m_1$ schreiben:

$$m_1 = \frac{V_1 \cdot p_1}{k} \qquad (13)$$

Daraus folgt:

$$\frac{V_1}{k} \cdot p_1(t) = \frac{V_1}{k} \cdot p_{1,0} - \int_{\tau=0}^{\tau=t} \frac{L \cdot D^3}{12 \, \eta \cdot l \cdot k} \cdot [p_1^2 - p_1 p_2] \, d\tau \qquad (14)$$

Die gesamte Gleichung lässt sich mit $V_1/k$ kürzen. Eine Differenzierung auf beiden Seiten liefert:

$$\frac{dp_1}{dt} = -\frac{L \cdot D^3}{12 \, V_1 \, \eta \, l} \cdot [p_1^2 - p_1 p_2] \qquad (15)$$

Es handelt sich hierbei um eine Bernoullische Differenzialgleichung von der Form:

$$x' = -a \cdot x \cdot (x - b), \qquad (16)$$

wobei

$$a = \frac{L \cdot D^3}{12 \, V_1 \, \eta \, l} \qquad (17)$$

$$b = p_2 \qquad (18)$$

Im Folgenden soll angenommen werden, dass der Ballon 8 erheblich größer ist als der Ballon 4:

$$V_2 \gg V_1$$

Daraus folgt, dass der Druck $p_2$ im Ballon 8 näherungsweise etwa konstant bleibt, auch wenn sich der Druck $p_1$ im Ballon 4 kurzzeitig ändert. Unter dieser Annahme sind die Koeffizienten a und b aus der Bernoullischen Differenzialgleichung (16) konstant, und die Lösung der Bernoullischen Differenzialgleichung lautet:

$$x(t) = \frac{b}{1 - e^{-abt - bc_1}} \qquad (19)$$

Die Integrationskonstante $c_1$ lässt sich wie folgt bestimmen:

$$p_1(t) = \frac{p_2}{1 - e^{-ap_2t - p_2c_1}} \qquad (20)$$

Für t = 0 muss gelten:

$$p_1(t) = p_{1,0} \qquad (21)$$

Daraus folgt:

$$p_{1,0} = \frac{p_2}{1 - e^{-p_2c_1}} \qquad (22)$$

$$\frac{p_2}{p_{1,0}} = 1 - e^{-p_2c_1} \qquad (23)$$

$$e^{-p_2c_1} = \frac{p_{1,0} - p_2}{p_{1,0}} \qquad (24)$$

$$-p_2c_1 = \ln \frac{p_{1,0} - p_2}{p_{1,0}} \qquad (25)$$

$$c_1 = -\frac{1}{p_2} \cdot \ln \frac{p_{1,0} - p_2}{p_{1,0}} = \frac{1}{p_2} \cdot \ln \frac{p_{1,0}}{p_{1,0} - p_2} \qquad (26)$$

Eingesetzt in Gleichung (2) liefert dies:

$$\frac{p_1(t)}{p_{1,0}} = \frac{p_2}{p_{1,0} - [p_{1,0} - p_2] \cdot e^{-(\pi R^4 p_2 t)/(8V_1\eta l)}} \qquad (27)$$

Diese Gleichung ist von der Form:

$$\frac{p_1(t)}{p_{1,0}} = \frac{p_2}{p_{1,0} - [p_{1,0} - p_2] \cdot e^{-t/\tau}} \qquad (28)$$

mit

$$\tau = (12 \cdot V_1 \cdot \eta \cdot l) / (L \cdot D^3 \cdot p_2) \qquad (29)$$

Für geringe Druckschwankungen im Ballon 4 gilt bspw.:

$$p_{1,0} \approx 0{,}9\, p_2$$

Ferner gilt für t = $\tau$:

$$e^{-t/\tau} = e^{-1} \approx 0{,}368 = k_1$$

Außerdem gilt für t = 2T:

$$e^{-t/\tau} = e^{-2} \approx 0{,}135 = k_2$$

Und für t = 4$\tau$:

$$e^{-t/\tau} = e^{-4} \approx 0{,}018 = k_4$$

In Gleichung (28) eingesetzt liefert dies:

$$\frac{p_1(t=v\tau)}{0{,}9\, p_2} = \frac{p_2}{0{,}9\, p_2 - (0{,}9\, p_2 - p_2) \cdot k_v}$$

bzw.:

$$\frac{p_1(t=v\tau)}{0{,}9\, p_2} = \frac{p_2}{(0{,}9 + 0{,}1 \cdot k_v) \cdot p_2}$$

Daraus folgt:

$$p_1(t=\tau) = p_2 \cdot \frac{0{,}9}{0{,}9 + 0{,}1 \cdot 0{,}368} \approx 0{,}96 \cdot p_2$$

Die nach t=$\tau$ noch verbleibende Regelabweichung von etwa $0{,}04 \cdot p_2$ entspricht 40 % der anfänglichen Abweichung von $0{,}10 \cdot p_2$.

$$p_1(t=2\tau) = p_2 \cdot \frac{0{,}9}{0{,}9 + 0{,}1 \cdot 0{,}135} \approx 0{,}98 \cdot p_2$$

Die nach t=2$\tau$ noch verbleibende Regelabweichung von etwa $0{,}02 \cdot p_2$ entspricht 20 % der anfänglichen Abweichung von $0{,}10 \cdot p_2$.

$$p_1(t=4\tau) = p_2 \cdot \frac{0{,}9}{0{,}9 + 0{,}1 \cdot 0{,}018} \approx 0{,}99 \cdot p_2$$

Die nach t=4$\tau$ noch verbleibende Regelabweichung von etwa $0{,}01 \cdot p_2$ entspricht 10 % der anfänglichen Abweichung von $0{,}10 \cdot p_2$.

Bei der Anwendung im Rahmen der thorakalen Atmung ist zu beachten, dass ein Atemzyklus etwa 3 sec. dauert. Damit in Verlauf eines thorakalen Atemzyklus der Cuff nicht undicht wird, sollte diese Ausgleichszeit $t_a = v\tau = 20$ ms sein, wobei man mit dem Parameter v wählen kann, wie gut die Ausregelung nach 20 ms sein soll.

Daraus ergibt sich $\tau = 20$ ms/v.

Das minimal anzustrebende Ergebnis ergibt sich für v = 1 und $t_a = 20$ ms wie folgt:

Daraus folgt:

$$\tau = 20 \cdot 10^{-3} \, s = \frac{12 \cdot 5 \cdot 10^{-6} \, m^3 \cdot 0{,}2 \, m \cdot 17{,}1 \cdot 10^{-6} \, Pas}{L \cdot D^3 \cdot 10^5 \, Pa}$$

Dabei wurde angenommen:

$$V_1 = 5 \, cm^3$$

$$l = 20 \, cm$$

$$p_2 = 10^5 \, Pa$$

Damit ergibt sich:

$$L \cdot D^3 = 10{,}26 \cdot 10^{-14} \, m^4,$$

Im folgenden soll weiter angenommen werden, dass in der Trachealröhre maximal eine lichte Öffnung mit einem Durchmesser von 10 mm zur Verfügung steht, also entsprechend einem Radius von 5 mm. Vernachlässigt man weiterhin die Mantelquerschnitte des Tubus 3 und des Ballons 4, so erstreckt sich das sekundäre Lumen maximal weit außen, und für den mittleren Radius $R_m$ kann man daher z.B. 4,8 mm annehmen. Daraus errechnet sich eine Umfangs-Länge $L_m = 2 \cdot \pi \cdot R_m$ von etwa 30 mm = $30 \cdot 10^{-3}$ m, und draus folgt:

$$D^3 = 10{,}26 \cdot 10^{-14} \, m^4 / L$$

bzw.:

$$D^3 = 102{,}6 \cdot 10^{-12} \, m^3 / 30$$

$$D^3 = 3{,}42 \cdot 10^{-12} \, m^3$$

$$D = 1{,}5 \cdot 10^{-4} \, m = 0{,}15 \, mm.$$

Damit hat das sekundäre Lumen eine Querschnittsfläche $Q_2$ von

$$Q_2 = L \cdot D = 30 \cdot mm \cdot 0{,}15 \; mm = 4{,}5 \; mm^2.$$

Für die Querschnittsfläche $Q_1$ des primären Lumens kann man D vom maximalen Radius der Trachealröhre von 5 mm abziehen und erhält dann 4,8 mm. Dies entspricht einer Querschnittsfläche $Q_1$ von

$$Q_1 = 4{,}85 \; mm \cdot 4{,}85 \; mm \cdot 3{,}14 = 74 \; mm^2.$$

Der freie Gesamtquerschnitt $Q = Q_1 + Q_2 = 78{,}5 \; mm^2$. Also gilt:

$$Q_2 / Q = Q_2 / (Q_1 + Q_2) = 4{,}5 / 78{,}5 = 0{,}06.$$

Fordert man eine kürzere Ausgleichszeit oder eine bessere Ausregelung innerhalb dieser Ausgleichszeit $t_a$, so ergeben sich strengere Anforderungen für das obige Verhältnis. Der Wert von 0,06 stellt demnach die absolute Untergrenze dar, die keinesfalls unterschritten werden sollte, weil solchenfalls eine Aspiration droht. Um eine Sicherheitsreserve zu haben, sollte man wenigstens wählen:

$$Q_2 / Q = Q_2 / (Q_1 + Q_2) \geq 0{,}08.$$

Außerdem wurde bei der obigen Berechnung die extrakorporale Zuleitung 7 ebenfalls vernachlässigt, die jedoch ebenfalls einen nicht unerheblichen Strömungswiderstand beisteuert. Daher wird empfohlen:

$$Q_2 / Q = Q_2 / (Q_1 + Q_2) \geq 0{,}10.$$

Setzt man andererseits $v = 4$ und $t_a = 10 \, ms$ (also die Forderung, dass nach 10 ms die restliche Regelabweichung kleiner als 10 % sein soll), so folgt:

$$\tau = 10 \; ms / 4 = 25 \cdot 10^{-4} \; s = \frac{12 \cdot 5 \cdot 10^{-6} \; m^3 \cdot 0{,}2 \; m \cdot 17{,}1 \cdot 10^{-6} \; Pas}{L \cdot D^3 \cdot 10^5 \; Pa}$$

Man erhält dann:

$$L \cdot D^3 = 82{,}08 \cdot 10^{-14} \; m^4,$$

$$D^3 = 82{,}08 \cdot 10^{-14} \; m^4 / L$$

bzw., mit $L = 30 \, mm$:

$$D^3 = 27{,}4 \cdot 10^{-12} \; m^3$$

$$D = 3 \cdot 10^{-4} \; m = 0{,}3 \; mm.$$

Damit hat das sekundäre Lumen eine Querschnittsfläche $Q_2$ von

$$Q_2 = L \cdot D = 30 \cdot mm \cdot 0{,}3 \; mm = 9 \; mm^2.$$

Für die Querschnittsfläche $Q_1$ des primären Lumens kann man D vom maximalen Radius der Trachealröhre von 5 mm abziehen und erhält dann 4,6 mm. Dies entspricht einer Querschnittsfläche $Q_1$ von

$$Q_1 = 4,6 \text{ mm} \cdot 4,6 \text{ mm} \cdot 3,14 = 66 \text{ mm}^2.$$

Der freie Gesamtquerschnitt $Q = Q_1 + Q_2 = 75 \text{ mm}^2$. Also gilt:

$$Q_2 / Q = Q_2 / (Q_1 + Q_2) = 9 / 75 = 0,12.$$

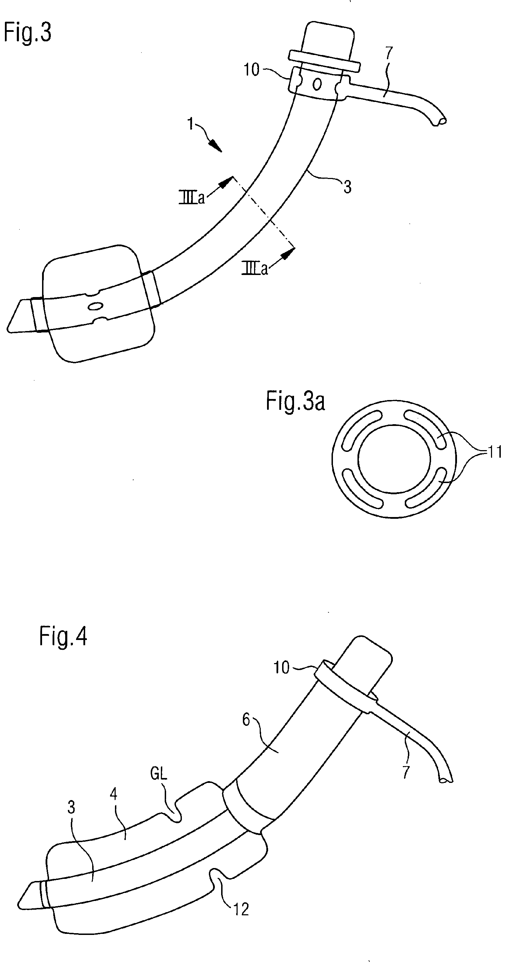

Fig. 3 beschreibt eine Ausführung des Schaftkörpers 3, der in die Schaftwandung integriert, ein oder mehrere volumenzuleitende Kanäle aufweist, die einen volumenverschiebenden Gesamtquerschnitt aufweisen, der hinsichtlich seiner Strömungsmechanik den in Fig. 2 dargestellten Verhältnisse entspricht. Der Schaftkörper besteht hier vorzugsweise aus mehr-lumig extrudiertem Schlauchmaterial, welches neben einem zentralen Lumen für die Beatmung, um das zentrale Lumen herum angeordnete Zuleitungslumina enthält. Bei einer solchen mehr-lumigen Ausführung können die Einzel-Lumina am proximalen Schaftende durch eine ringartig umlaufende Struktur 10 gebündelt bzw. zusammengeführt werden.

Fig. 3a zeigt einen bespielhaften Schaftquerschnitt mit mehr-lumig ausgeführten Volumenzuleitungen 11.

Fig. 4 zeigt eine Ausführungsvariante, bei der das tracheal dichtende Ballonsegment 4 nach proximal bis zur, oder über die Stimmlippenebene GL hinaus verlängert ist. Diese Ausführung, bei der das so verlängerte Ballonsegment anteilig aus dem Thorax herausragt, und somit nicht den thorakalen Druckschwankungen exponiert ist, ermöglicht ein besonders großes Ballonvolumen, welches in der Lage ist, eine druckerhaltende Pufferwirkung zu entwickeln, wenn es im distalen, tracheal platzierten Segment des Ballons zu einer atemmechanisch bedingten Reduktion der transmuralen Kraft auf den Ballon kommt. Die so ermöglichte Volumenreserve ist auch dann partiell puffernd wirksam, wenn keine externe volumenkompensierende Einheit mit dem Trachealtubus verbunden ist.

Durch die große Kontaktfläche zur exponierten trachealen, glottischen und supraglottischen Schleimhaut wird zudem ein maximal verlängerter Migrationsweg für Sekrete bzw. darin enthaltene Erreger geschaffen.

Zur Erleichterung der trans-glottischen Positionierung des Tubus, kann der Ballon im Bereich der Stimmbänder GL mit einer zirkulären Einschnürung 12 versehen sein. Diese Einschnürung ermöglicht zudem die freie Beweglichkeit der Stimmlippen, weitgehend unabhängig vom herrschenden Ballon-Fülldruck.

Der distale Schaftanteil ist bevorzugt als dünnwandiger, einlumiger, durch eine Korrugation der Schaftwandung stabilisierter Schlauch ausgeführt. Der distale Schaft geht nach proximal in einen Schaftanteil 6 über, der, wie in Fig. 1 beschrieben, eine großlumige Zuleitung zum proximalen Ballonsegment 5 erlaubt. Der Schaft 6 weist in bevorzugter Ausführung, wie in Fig. 3a beschrieben, einen Aufbau mit mehrlumigem Profil auf. Das mehrlumige Schaftsegment 6 ist derart stabil ausgeführt, dass es einen Beißschutz darstellt, was einen lumenverschließende Verschluss des Beatmungslumens verhindert.

Durch ein am proximalen Schlauchende abschließendes Element 10 können die im Schaft 6 integrierten, zuleitenden Lumina gebündelt werden. Die Anbindung des Abschlusselementes 7 an ein Reservoir erfolgt wiederum ausreichend großlumig.

Der dünnwandige, ein-lumig ausgeführte Schaftkörper 3 wird zu Stabilisierung des Schaftlumens und zur Ermöglichung einer weitgehend spannungslosen axialen Biegung des Schaftes mit einer welligen Korrugation ausgestattet. In der bevorzugten Ausführung sollten so axiale Biegungen des Schaftes von 90 bis 135 Grad ohne relevante Lumeneinengung und ohne relevante auf die Gewebe wirkende elastische Rückstellkräfte möglich sein.

Bei Innendurchmessern des Schaftes von 7 bis 10 mm kann beispielsweise bei der Kombination einer Wandungsstärke von ca. 0,4 mm, einer Shore Härte von 95A, einem Peak-tot-Peak Wellungsabstand von 0,5 mm sowie einer Wellungsamplitude von 0,75 mm ein entsprechend knickstabiler, lumen- bzw. flussoptimierter Schaft hergestellt werden.

Im Falle der korrugierten Ausführung des Schaftes 3 kann bei Verwendung einer wechselbaren Innenkanüle, wie diese bei Tracheostomiekanülen üblich sind, eine Innennkanüle mit kongruent korrugiertem Profil verwendet werden, deren Wellung sich in die Wellung der Außenkanüle optimal einfügt und die Außenkanüle vorteilhaft stabilisiert.

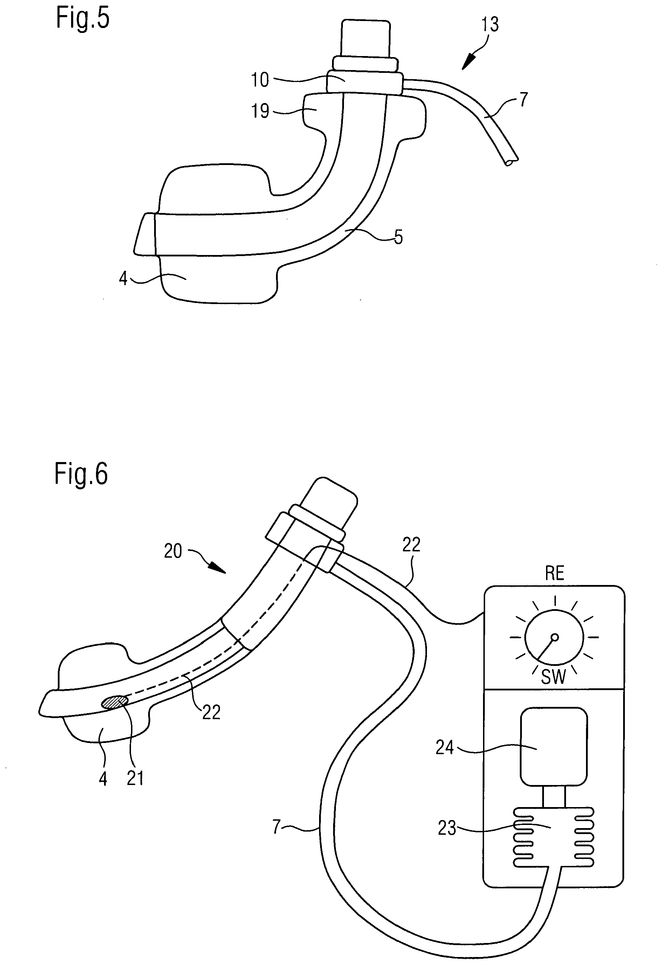

Fig. 5 zeigt eine beispielhafte Anwendung der fluss-optimierten Ausführung des zuleitenden Lumens zum tracheal dichtenden Ballonelement 4 bei einer Tracheostomiekanüle 13. In analoger Weise zur Gestaltung bei Trachealtuben, wird das volumenzuleitende Ballonende 5 hier durch das chirurgisch angelegte Stoma zur Luftröhre geleitet und auf einem Konnektor 10 unterhalb des Kanülenflansches aufgebracht. Die Querschnittfläche G des zuleitenden Endes 5 kann so gewählt sein, dass es, über die erfindungsgemäßen Erfordernisse des raschen Volumenflusses hinaus, zur Dichtung des Stomas geeignet ist und somit den Austritt von Sekreten verhindert. Das proximale Ballonende 19 kann zudem vorteilhaft als wulstartige Erweiterung ausgebildet sein, die sich direkt unterhalb des Kanülenflansches dichtend auf das Stoma legt.

Fig. 6 zeigt einen Trachealtubus 20, der im Bereich des tracheal dichtenden Ballonsegmentes 4 mit einem druckaufnehmenden bzw. druckmessenden Fühlerelement 21 versehen ist. Der Druckfühler ist in bevorzugter Ausführungsweise ein elektronisches Bauelement, welches sein Messsignal über eine Kabelleitung 22 an einen elektronisch gesteuerten Regler RE weiterleitet. Das Fühlerelement besteht vorzugsweise aus einem Absolutdrucksensor. Es können vorzugsweise Fühler auf der Basis von Dehnungsmessstreifen oder piezzo-elektrischen Sensoren verwendet werden. Der Regler RE weist beispielsweise ein balgartiges oder kolbenartiges Reservoir 23 auf, welches durch einen Antrieb 24 betätigt, entweder Volumen zum Ballon 4 hin verschiebt, oder aus dem Ballon 4 entnimmt, der Antrieb kann beispielsweise aus einem Schrittmotor bestehen oder als linear magnetischer Antrieb aufgebaut sein. Die Steuerung des Reglers RE ist derart ausgelegt, dass Abweichungen des Fülldrucks im Bereich des dichtenden Ballonsegmentes 4 sofort durch eine entsprechende Volumenverschiebung kompensiert werden bzw. der Fülldruck auf einem am Regler einstellbaren Sollwert SW konstant gehalten wird. Die Stabilisierung des dichtenden Ballondrucks erfolgt mit diesem Verfahren bereits zu einem Zeitpunkt, der vor dem Einsetzen eines maschinellen Beatmungshubes bzw. einem tatsächlichen Volumenfluss von Atemgas in die Lunge des Patienten liegt. Dies ist besonders bei Patienten relevant, die beispielsweise nach langer kontrollierter maschineller Beatmung vermehrte Atemarbeit aufwenden müssen, um eine nicht ausreichend elastische Lunge im Thorax bis zu einem Punkt aufzuspannen, der einen lungenwärts gerichteten Volumenstrom von Atemluft auslöst.

In dieser Phase der "isometrischen" Anspannung der Lunge innerhalb des Thorax und des damit einhergehenden Druckabfalls innerhalb des Brustkorbs kann es zu dichtungskritischen Abfällen des Ballonfülldrucks kommen. Auch bei klinisch apnoeischen Patienten, also Patienten, die zwar (isometrische) Atemarbeit leisten, aber keinen wahrnehmbaren Atemgasstrom erzeugen, kann mit der beschriebenen Sensortechnologie eine aspirationspräventive Intubation gewährleistet werden. Kommt es zu plötzlichen Druckschwankungen im Ballon, wie z. B. durch Lagewechsel des Patienten oder Hustenattacken verursacht, kann der beschriebene Regelkreis ebenfalls effizient schnell Volumen zum dichtenden Ballon hin verschieben, oder aus diesem entnehmen.

Im Gegensatz zu einem regelnden Reservoir 2, wie in Fig. 1 beschrieben, welcher ein kompensierendes Reservevolumen unter einem isobaren Druck von vorzugsweise 20 bis 35 mbar zur Verfügung stellt, kann bei der elektronischen Regelung innerhalb des druckerzeugenden Elementes 22 des Reglers RE ein Druck aufgebaut werden, der den tracheal unkritischen Dichtungsdruck von 20 bis 35 mbar kurzzeitig überschreitet und so durch einen entsprechenden passageren Druckgradienten den Volumenfluss zum dichtenden Ballon hin beschleunigt. Die kontinuierliche Messfunktion des Fühlers stellt dabei sicher, dass keine kritischen Druckwerte im Ballon erreicht werden.

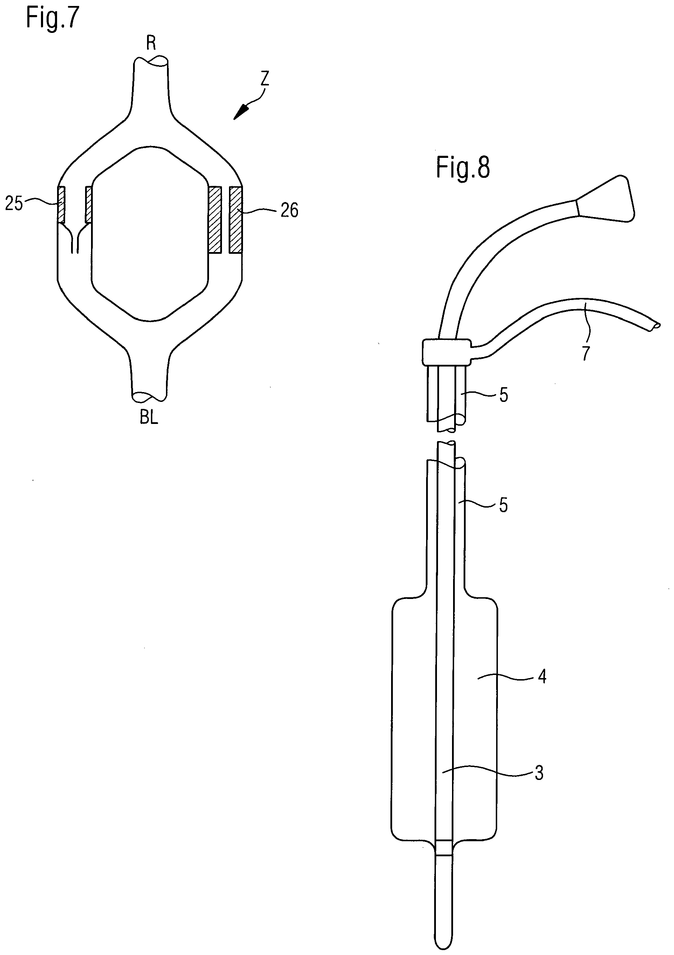

Fig. 7. Um größere, dichtungskritische Entleerungen des trachealen Ballonsegmentes bzw. Ballons in das Reservoir zu vermeiden, wie diese beispielsweise beim Husten oder Pressen des Patienten erfolgen können, kann die verbindende Zuleitung Z zwischen Ballon und Regler mit einem großlumigen, flußrichtenden Ventil 25 ausgestattet sein, welches den Rückschlag von Füllmedium vom Ballon BL zum Reservoir R verhindert. Dazu parallel ist ein nichtflußrichtendes Drosselelement 26 angeordnet, dass den langsamen Volumenausgleich zwischen Ballon und Reservoir ermöglicht.

Fig. 8 zeigt eine analoge Anwendung der beschriebenen dynamischen Tamponade, die den dichtenden Verschluss der Speiseröhre eines Patienten mittels eines befüllbaren Ballonelementes ermöglicht. Das dichtende Ballonsegment 4 geht in eine nach proximal verlängerte Verjüngung 5 über die zum Schaftelement 3 hin ein freier Spaltraum S zur fluss-effizienten Verschiebung eines Füllmediums definiert. Die proximale Verlängerung 5 des Ballonkorpus reicht optional bis auf die Höhe der Mund- oder auch der Nasenplatzierung, bzw. reicht über die jeweilige Öffnung hinaus. Die Verlängerung 5 geht in eine fluss-effizient dimensionierte Schlauchleitung 7 über, die wiederum an ein erfindungsgemäßes Reservoir gekoppelt ist oder mit einem anderen erfindungsgemäßen Regelmechanismus verbunden ist.

Mit den in den vorausgehenden Figuren beschriebenen Vorrichtungen zur fluss-optimierten Volumenverschiebung zwischen einem tracheal oder ösophageal dichtenden Ballon 4 und einem extrakorporal regelnden Reservoir 2, können dichtungswirksame Volumenkompensationen innerhalb eines treachealen oder ösophagealen Ballonkörpers innerhalb von 10 bis 30 Millisekunden, bevorzugt innerhalb von 10 bis 15 Millisekunden nach Beginn einer intra-thorakalen Druckänderung erfolgen.

Bezugszeichenliste

| | | | |
|---|---|---|---|
| 1 | Vorrichtung | ID | Innenquerschnitt des Tubus |
| 2 | Reservoir | OD | Außenquerschnitt des Tubus |
| 3 | Tubus | GL | Stimmlippenebene |
| 4 | Ballon | RE | Regler |
| 5 | proxim., verjüngtes Ballonende | SW | Sollwert |
| 6 | proximales Schaftelement | BL | Ballon |

(fortgesetzt)

| 7 | Extrakorporale Zuleitung | R | Reservoir |
|---|---|---|---|
| 8 | Reservoir-Volumen | | |
| 9a | distales Schlauchende | | |
| 9b | proximales Schlauchende | | |
| 10 | ringartige Struktur | | |
| 11 | Volumenzuleitung | | |
| 12 | Einschnürung | | |
| 13 | Tracheostomiekanüle | | |
| 19 | proximales Ballonende | | |
| 20 | Trachealtubus | | |
| 21 | Fühlerelement | | |
| 22 | Kabelleitung | | |
| 23 | Reservoir | | |
| 24 | Antrieb | | |
| 25 | flussaufrichtendes Ventil | | |
| 26 | Drosselelement | | |
| K | Kraft | | |
| G | Querschnittsfläche | | |
| S | Spaltraum | | |

**Patentansprüche**

1. Vorrichtung (1) zur dynamisch dichtenden Intubation eines Hohlorgans, umfassend einen in das Hohlorgan einführbaren Tubus (3) mit einem primären Lumen als Zugang durch das oder zu dem betreffende(n) Hohlorgan, sowie einen diesen Tubus (3) zwecks Abdichtung gegenüber dem Hohlorgan manschettenförmig umgebenden Ballon (4) mit wenigstens einem oder mehreren sekundären Lumen (Lumina) zur Befüllung des Ballons (4), wobei innerhalb jeder von der lokalen Längsrichtung der Vorrichtung lotrecht durchsetzten Querschnittsebene für die Querschnittsfläche des lichten Gesamtquerschnitts Q1 des primären Lumens und die Summe $Q_2$ der Querschnittsflächen der lichten Querschnitte aller sekundären Lumina gilt:

$$Q_2 / (Q_1 + Q_2) \geq 0{,}06,$$

   **dadurch gekennzeichnet, dass** an dem proximalen Ende des Tubus (3) ein mit allen sekundären Lumina kommunizierender Anschluss für einen extrakorporalen Befüllungsschlauch (7) vorgesehen ist, und wobei in einem mit den sekundären Lumina kommunizierenden Befüllungsschlauch (7)

   a) ein Einwegeventil (25) angeordnet ist, welches bei einem Druckgefälle von dem extrakorporalen Vorratsballon (8) in Richtung zu dem intrakorporalen Ballon (4) eine Strömung zulässt, in der umgekehrten Richtung jedoch nicht, sowie
   b) eine Strömungsdrossel (26) angeordnet ist, welche in jeder Strömungsrichtung nur eine begrenzte Strömung zulässt,

   wobei das Einwegeventil (25) und die Strömungsdrossel (26) parallel geschalten sind.

2. Vorrichtung (1) nach Anspruch 1, **dadurch gekennzeichnet, dass** innerhalb jeder von der lokalen Längsrichtung der Vorrichtung (1) lotrecht durchsetzten Querschnittsebene für die Querschnittsfläche des lichten Gesamtquerschnitts Q1 des primären Lumens und die Summe $Q_2$ der Querschnittsflächen der lichten Querschnitte aller sekundären Lumina gilt:

$$Q_2 / (Q_1 + Q_2) \geq 0{,}08,$$

vorzugsweise:

$$Q_2 / (Q_1+Q_2) \geq 0{,}10{,}$$

insbesondere:

$$Q_2 / (Q_1+Q_2) \geq 0{,}12{.}$$

3. Vorrichtung (1) nach Anspruch 1 oder 2, **dadurch gekennzeichnet, dass** der Ballon (4) einen radial erweiterten, distalen Bereich (4) zur Abdichtung aufweist sowie einen sich daran anschließenden und demgegenüber radial verjüngten, proximalen Bereich (5) als Umhüllung des (der) sekundären Lumens (Lumina) zur Befüllung des distalen Abdichtungs-Bereichs (4).

4. Vorrichtung (1) nach einem der Ansprüche 1 bis 3, **dadurch gekennzeichnet, dass** der Ballon (4) mit unterschiedlichen Außendurchmessern in seinem distalen und seinem proximalen Bereich (5) präformiert ist.

5. Vorrichtung (1) nach einem der vorhergehenden Ansprüche, **dadurch gekennzeichnet, dass** in dem proximalen Bereich des Ballons (4) ein einziges sekundäres Lumen vorgesehen ist, welches das primäre Lumen konzentrisch außen umgibt.

6. Vorrichtung (1) nach einem der vorhergehenden Ansprüche, **dadurch gekennzeichnet, dass** der proximale Bereich (5) des Ballons (4) sich nicht bis zu dem proximalen Ende des Tubus (3) erstreckt, sondern vorher endet.

7. Vorrichtung (1) nach einem der vorhergehenden Ansprüche, **dadurch gekennzeichnet, dass** der Ballon (4) oder der proximale Bereich des Ballons (4) an einer Stirnseite eines schlauchförmigen Elements (6) endet, in welchem sich das primäre Lumen als lichte Öffnung radial innerhalb des Schlauchmantels fortsetzt, während das sekundäre Lumen in Form eines oder mehrerer in den Schlauchmantel selbst eingeformter Kanäle (11) fortsetzt.

8. Vorrichtung (1) nach Anspruch 7, **dadurch gekennzeichnet, dass** der minimale Gesamtquerschnitt aller in den Schlauchmantel als sekundäre Lumina eingeformten Kanäle (11) gleich oder größer ist als der maximale Querschnitt des ringförmigen sekundären Lumens im proximalen Bereich des Ballons (4).

9. Vorrichtung (1) nach einem der vorhergehenden Ansprüche, **dadurch gekennzeichnet, dass** sich im Bereich des proximalen Endes des Tubus (3) ein ringförmiger Sammelkanal (10) befindet, mit welchem alle sekundären Lumina kommunizieren, insbesondere alle in den Schlauchmantel als sekundäre Lumina eingeformten Kanäle (11).

10. Vorrichtung (1) nach einem der vorhergehenden Ansprüche, **dadurch gekennzeichnet, dass** mit dem an einem mit allen sekundären Lumina kommunizierenden Befüllungsschlauch (7) ein extrakorporaler Vorratsballon (8) verbunden oder verbindbar ist.

11. Vorrichtung (1) nach Anspruch 10, **dadurch gekennzeichnet, dass** der extrakorporale Vorratsballon (8) in frei entfaltetem Zustand ein größeres Volumen aufweist als der intrakorporale, manschettenförmige Dichtungsballon (4) im distalen Bereich des Tubus (3).

12. Vorrichtung (1) nach Anspruch 10 oder 11, **dadurch gekennzeichnet, dass** der extrakorporale Vorratsballon (8) mit einem konstanten oder näherungsweise konstanten Druck beaufschlagt wird, beispielsweise durch ein Gewicht oder ein Federelement.

13. Vorrichtung (1) nach Anspruch 10 oder 11, **dadurch gekennzeichnet, dass** der Druck in dem extrakorporalen Vorratsballon (8) aktiv gesteuert oder geregelt wird.

14. Vorrichtung (1) nach Anspruch 10 oder 11, **dadurch gekennzeichnet, dass** der Druck in dem extrakorporalen Vorratsballon (8) derart aktiv geregelt wird, dass der Druck in dem intrakorporalen, manschettenförmigen Dichtungsballon (4) konstant gehalten wird, insbesondere wobei der Druck in dem intrakorporalen manschettenförmigen Dichtungsballon (4) gemessen und als Istwert für einen Regelkreis dient, der auf den Druck in dem extrakorporalen Vorratsballon (8) einwirkt.

15. Vorrichtung (1) nach einem der vorhergehenden Ansprüche, **dadurch gekennzeichnet, dass** der zuleitende Füllkanal eine flusswirksame Querschnittsfläche aufweist, die einer kreisrunden Querschnittsfläche bei einem Kreisdurchmesser von mindestens 2 mm entspricht, und/oder die einer kreisrunden Querschnittsfläche bei einem Kreisdurchmesser von 4 bis 6 mm entspricht.

**Claims**

1. Device (1) for the dynamically sealing intubation of a hollow organ, comprising a tubus (3) insertable into the hollow organ with a primary lumen as an entrance through or to the regarding hollow organ, and with a balloon (4) encompassing this tubus (3) like a sleeve for the purpose of sealing, with at least one or several secondary lumen (lumina) for filling the balloon (4), wherein it applies for the cross-sectional area of the clear cross-section $Q_1$ of the primary lumen and for the sum $Q_2$ of the cross-sectional areas of the clear cross-sections of all secundary lumina within each cross-sectional plane penetrated perpendicularly by the local longitudinal direction of the device:

$$Q_2 / (Q_1 + Q_2) \geq 0{,}06,$$

**characterized in that** a connection for an extracorporal filling tube (7), which connection communicates with all secundary lumina, is provided at the proximal end of the tubus (3), and wherein, in a filling tube (7) communicating with the secundary lumina,

a) a one-way valve (25) is arranged which permits a flow in case of a pressure gradient from the extracorporeal supply balloon in a direction to the intracorporeal balloon (4), but not in the opposite direction, and
b) a flow throttle (26) is arranged which permits only a limited flow in each direction of flow,

wherein the one-way valve (25) and the flow throttle (26) are connected in parallel.

2. Device (1) according to claim 1, **characterized in that** within each cross-sectional plane penetrated perpendicularly by the local longitudinal direction of the device, it applies for the cross-sectional area of the clear cross-section $Q_1$ of the primary lumen and for the sum $Q_2$ of the cross-sectional areas of the clear cross-sections of all secundary lumina:

$$Q_2 / (Q_1 + Q_2) \geq 0{,}08,$$

preferably:

$$Q_2 / (Q_1 + Q_2) \geq 0{,}10,$$

especially:

$$Q_2 / (Q_1 + Q_2) \geq 0{,}12.$$

3. Device (1) according to claim 1 or 2, **characterized in that** the balloon (4) comprises a radially extended distal area (4) for the purpose of sealing and an adjacent, radially tapered proximal area (5) as an envelope of the secundary lumen (lumina) for filling the distal sealing area (4).

4. Device (1) according to one of claims 1 to 3, **characterized in that** the balloon (4) is preformed with different outer diameters in its distal and its proximal area (5).

5. Device (1) according to one of the preceding claims, **characterized in that** there is a single secundary lumen in the proximal area of the balloon (4), which concentrically surrounds the primary lumen at its outer side.

6. Device (1) according to one of the preceding claims, **characterized in that** the proximal area (5) of the balloon (4) does not extend up to the proximal end of the tubus (3), but terminates in front of that.

7. Device (1) according to one of the preceding claims, **characterized in that** the balloon (4) or the proximal area of the balloon (4) terminates at a front side of a hose-like element (6) wherein the primary lumen extends as a clear opening radially within the jacket of the hose, while the secundary lumen extends in form of one or more channels (11) formed into the jacket of the hose itself.

8. Device (1) according to claim 7, **characterized in that** the minimum overall cross-section of all channels (11) formed into the jacket of the hose as secundary lumina is equal to or greater than the maximum cross-section of the annular secundary lumen in the proximal area of the balloon (4).

9. Device (1) according to one of the preceding claims, **characterized in that** an annular collective channel (10) is located in the area of the proximal end of the tubus (3), with which all secundary lumina correspond, especially all channels (11) formed into the jacket of the hose as secundary lumina.

10. Device (1) according to one of the preceding claims, **characterized in that** an extracorporeal supply balloon (8) is connected or connectable to a filling hose (7) communicating with all secundary lumina.

11. Device (1) according to claim 10, **characterized in that** the extracorporeal supply balloon (8) comprises a greater volume in the freely unfolded state than the intracorporeal cuff-like sealing balloon (4) in the distal area of the tubus (3).

12. Device (1) according to claim 10 or 11, **characterized in that** the extracorporeal supply balloon (8) is biased with a constant or nearly constant pressure, for example by a weight or by a spring element.

13. Device (1) according to claim 10 or 11, **characterized in that** the pressure in the extracorporeal supply balloon (8) is actively steered or controlled.

14. Device (1) according to claim 10 or 11, **characterized in that** the pressure in the extracorporeal supply balloon (8) is actively controlled in such a way that the pressure in the intracorporeal cuff-like sealing balloon (4) is kept constant, especially wherein the pressure in the intracorporeal cuff-like sealing balloon (4) is measured and serves as an actual value for a control circuit which acts upon the pressure in the extracorporeal supply balloon (8).

15. Device (1) according to one of the preceding claims, **characterized in that** the feeding filling channel comprises a flow-effective cross-sectional area, which corresponds to a circular cross-sectional area at a circle diameter of at least 2 mm, and/or which corresponds to a circular cross-sectional area at a circle diameter of 4 to 6 mm.

**Revendications**

1. Dispositif (1) permettant de réaliser l'intubation d'un organe creux en assurant une étanchéité dynamique, comprenant un tube (3) pouvant être introduit dans l'organe creux avec une lumière primaire comme accès par le ou à l'organe creux correspondant, ainsi qu'un ballonnet (4) en forme de manchette entourant ce tube (3) à des fins d'étanchement par rapport à l'organe creux avec au moins une ou plusieurs lumières secondaires pour le remplissage du ballonnet (4), en ce que dans chaque plan transversal traversé à la verticale par le sens longitudinal local du dispositif pour la surface de section de la section transversale totale intérieure $Q_1$ de la lumière primaire et la somme $Q_2$ des surfaces de section des sections transversales intérieures de toutes les lumières secondaires s'applique :

$$Q_2 / (Q_1 + Q_2) \geq 0{,}06,$$

**caractérisé en ce que,** au niveau de l'extrémité proximale du tube (3) est prévu un raccordement communiquant avec toutes les lumières secondaires pour un tuyau flexible de remplissage (7) extracorporel, et **en ce que** dans un tuyau flexible de remplissage (7) communiquant avec les lumières secondaires

a) est disposée une valve unidirectionnelle (25) qui, en cas de chute de pression, permet un écoulement du ballonnet de réserve (8) extracorporel dans le sens du ballonnet (4) intracorporel, toutefois pas dans le sens inverse, ainsi qu'

b) est disposé un étranglement d'écoulement (26), lequel permet seulement un écoulement limité dans chaque sens d'écoulement,

**en ce que** la valve unidirectionnelle (25) et l'étranglement d'écoulement (26) sont montés en parallèle.

2. Dispositif (1) selon la revendication 1, **caractérisé en ce que** dans chaque plan transversal traversé à la verticale par le sens longitudinal local du dispositif (1) pour la surface de section de la section transversale totale intérieure $Q_1$ de la lumière primaire et la somme $Q_2$ des surfaces de section des sections transversales intérieures de toutes les lumières secondaires s'applique :

$$Q_2 / (Q_1+Q_2) \geq 0,08,$$

de préférence :

$$Q_2 / (Q_1+Q_2) \geq 0,10,$$

en particulier :

$$Q_2 / (Q_1+Q_2) \geq 0,12.$$

3. Dispositif (1) selon la revendication 1 ou 2, **caractérisé en ce que** le ballonnet (4) présente une zone distale radialement élargie (4) pour l'étanchement ainsi qu'une zone proximale qui y est adjacente et radialement rétrécie (5) par rapport à celle-ci comme enveloppe de la/des lumière(s) secondaire(s) pour le remplissage de la zone d'étanchement distale (4).

4. Dispositif (1) selon l'une des revendications 1 à 3, caractérisé en ce que le ballonnet (4) est pré-façonné avec différents diamètres extérieurs dans sa zone distale et sa zone proximale (5).

5. Dispositif (1) selon l'une des revendications précédentes, **caractérisé en ce qu'**une seule et unique lumière secondaire est prévue dans la zone proximale du ballonnet (4), ladite seule et unique lumière secondaire entourant concentriquement à l'extérieur la lumière primaire.

6. Dispositif (1) selon l'une des revendications précédentes, **caractérisé en ce que** la zone proximale (5) du ballonnet (4) ne s'étend pas jusqu'à l'extrémité proximale du tube (3), mais se termine avant.

7. Dispositif (1) selon l'une des revendications précédentes, **caractérisé en ce que** le ballonnet (4) ou la zone proximale du ballonnet (4) se termine au niveau d'une face frontale d'un élément en forme de tuyau flexible (6), dans lequel la lumière primaire continue en tant qu'ouverture intérieure radialement dans la gaine de tuyau flexible tandis que la lumière secondaire continue sous forme d'un ou de plusieurs canaux auto-moulés (11) dans la gaine de tuyau flexible.

8. Dispositif (1) selon la revendication 7, **caractérisé en ce que** la section transversale totale minimale de tous les canaux moulés (11) comme lumières secondaires dans la gaine de tuyau flexible est égale ou supérieure à la section transversale maximale de la lumière secondaire annulaire dans la zone proximale du ballonnet (4).

9. Dispositif (1) selon l'une des revendications précédentes, **caractérisé en ce qu'**un canal collecteur annulaire (10) se trouve dans la zone de l'extrémité proximale du tube (3), canal avec lequel toutes les lumières secondaires communiquent, en particulier tous les canaux moulés (11) en tant que lumières secondaires dans la gaine de tuyau flexible.

**10.** Dispositif (1) selon l'une des revendications précédentes, **caractérisé en ce qu'**un ballonnet de réserve (8) extracorporel est raccordé ou peut être raccordé à un tuyau flexible de remplissage (7) communiquant avec toutes les lumières secondaires.

**11.** Dispositif (1) selon la revendication 10, **caractérisé en ce que** le ballonnet de réserve extracorporel (8) présente à l'état librement déployé un plus grand volume que le ballonnet d'étanchéité intracorporel en forme de manchette (4) dans la zone distale du tube (3).

**12.** Dispositif (1) selon la revendication 10 ou 11, caractérisé en ce quele ballonnet de réserve extracorporel (8) est soumis à une pression constante ou approximativement constante, par exemple par un poids ou un élément à ressort.

**13.** Dispositif (1) selon la revendication 10 ou 11, **caractérisé en ce que** la pression dans le ballonnet de réserve extracorporel (8) est commandée ou réglée activement.

**14.** Dispositif (1) selon la revendication 10 ou 11, **caractérisé en ce que** la pression dans le ballonnet de réserve extracorporel (8) est réglée activement de façon à ce que la pression dans le ballon d'étanchéité intracorporel en forme de manchette (4) soit maintenue constante, en particulier **en ce que** la pression dans le ballonnet d'étanchéité intracorporel en forme de manchette (4) est mesurée et sert de valeur réelle pour un circuit de régulation qui agit sur la pression dans le ballonnet de réserve extracorporel (8).

**15.** Dispositif (1) selon l'une des revendications précédentes, **caractérisé en ce que** lecanal de remplissage d'alimentation présente une surface de section active d'écoulement qui correspond à une surface de section circulaire d'un diamètre de cercle d'au moins 2 mm, et/ou qui correspond à une surface de section circulaire d'un diamètre de cercle de 4 à 6 mm.

Fig.1

Fig.2

**Fig.3**

**Fig.3a**

**Fig.4**

Fig.5

Fig.6

## Fig.7

## Fig.8

**IN DER BESCHREIBUNG AUFGEFÜHRTE DOKUMENTE**

**In der Beschreibung aufgeführte Patentdokumente**

- US 4762129 A
- US 213146062 A1
- US 5029591 A
- US 2007277830 A1
- EP 2013056169 W

**In der Beschreibung aufgeführte Nicht-Patentliteratur**

- **SIEHE HIERZU ; BADENHORST CH.** Changes in tracheal cuff pressure in respiratory support. *Crit-CareMed,* 1987, vol. 15/4, 300-302
- **SIEHE BASSI GL.** *CritCareMed,* 2013, vol. 41, 518-526